(19) Europäisches Patentamt
European Patent Office
Office européen des brevets

(11) Publication number: 0 170 003
A1

(12) # EUROPEAN PATENT APPLICATION

(21) Application number: 85106706.6

(22) Date of filing: 02.02.83

(51) Int. Cl.[4]: C 01 B 33/28
B 01 J 29/00

(30) Priority: 05.02.82 JP 16395/82

(43) Date of publication of application:
05.02.86 Bulletin 86/6

(84) Designated Contracting States:
DE FR GB IT NL

(60) Publication number of the earlier application in accordance with Art. 76 EPC: 0 087 017

(71) Applicant: RESEARCH ASSOCIATION FOR PETROLEUM ALTERNATIVE DEVELOPMENT
4-2, 1-chome Uchikanda
Chiyoda-ku Tokyo(JP)

(72) Inventor: Kozo, Takatsu
No. 1660, Kamiizumi Sodegaura-machi
Kimitsu-gun Chiba-ken(JP)

(72) Inventor: Noboru, Kawata
No. 11-7, 1-chome, Sakuradai
Ichihara-shi Chiba-ken(JP)

(74) Representative: Türk, Dietmar, Dr. rer. nat et al,
Redies, Redies, Türk & Gille Patentanwälte
Brucknerstrasse 20
D-4000 Düsseldorf 13(DE)

(54) **Crystalline silicates.**

(57) A novel crystalline silicate is described, which has a composition represented by the general formula (I) after being calcined in air at 550°C,

$$p(M_{2/n}O) \cdot (Al_2O_3) \cdot q(SiO_2) \text{ ----- (I)}$$

(wherein M represents an alkali metal and/or alkaline earth metal, n represents the atomic valency of M, and p and q represent molar ratios and are $0.3 \leqq p \leqq 3.0$, and $q \geqq 10$), and a X-ray diffraction pattern as described in the appended claim. The novel crystalline silicate can be used as a catalyst in various chemical reactions, e.g., in the production of para-xylene by the methylation of toluene, and in the production of an aromatic component-rich gasoline fraction from those hydrocarbons having a low aromatic component content.

EP 0 170 003 A1

# CRYSTALLINE SILICATES

## BACKGROUND OF THE INVENTION

The present invention relates to crystalline silicates. More particularly, the present invention is concerned with crystalline silicates having a novel structure, which can be used as catalysts for various chemical reactions with satisfactory results.

A number of crystalline silicates, natural or synthetic, have heretofore been known, and a wide variety of methods for producing crystalline silicates have been proposed. These methods are usually carried out in aqueous solutions. Recently, however, there have been developed methods in which the reaction is performed in aqueous solutions with alcohols added thereto (see U.S. Patent 4,199,556 and Japanese Patent Application Laid-Open No. 43800/77). Crystalline silicates produced by the conventional methods have the known crystal structures, e.g., ZSM-5 and Zeta-3.

## SUMMARY OF THE INVENTION

As a result of extensive investigations to develop crystalline silicate having novel compositions and crystal structures, it has been found that the addition of a large amount of methanol to starting materials leads to the production of crystalline silicate having a crystal structure which is different from the known ones, e.g., ZSM-5 and Zeta-3.

The present invention provides:

(1) a crystalline silicate (named as "ISI-1") having a composition represented by the general formula (I) as described hereinafter and main X-ray diffraction data as shown in Table 1 (both being determined after calcining in air at 550°C); and

(2) a process for producing a crystalline silicate having a composition represented by the general formula (I) as described hereinafter and main X-ray diffraction data as shown in Table 1 (both being determined after calcining in air at 550°C) which comprises reacting an aqueous mixture containing (a) a silica source, (b) an alumina source, (c) an alkali metal and/or alkaline earth metal source, and (d) methanol in the following molar ratios:

silica/alumina $\geq$ 10/1
methanol/water = 0.1/1 to 10/1
methanol/silica = 5/1 to 100/1
hydroxyl group/silica = 0.01/1 to 0.5/1
alkali metal and/or alkaline
earth metal/silica = 0.1/1 to 3/1
~~hydroxyl group/alumina $\geq$ 100/1~~

at a temperature of from 100 to 300°C until the desired crystalline silicate is formed.

General Formula (I)

$$p(M_{2/n}O)\cdot(Al_2O_3)\cdot q(SiO_2)$$

wherein M represents an alkali metal and/or alkaline earth metal, n represents the atomic valency of M, and p and q represent molar ratios and are:

$0.3 \leq p \leq 3.0$, and $q \geq 10$.

Table 1

| Lattice Spacing d (Å) | Relative Intensity |
|---|---|
| 10.89 ± 0.2 | strong |
| 8.74 ± 0.2 | medium |
| 6.94 ± 0.15 | medium |
| 4.37 ± 0.1 | very strong |
| 3.68 ± 0.1 | very strong |
| 3.62 ± 0.07 | strong |
| 3.46 ± 0.07 | strong |
| 2.52 ± 0.05 | medium |

Irradiation: Cu - Kα

Wavelength: 1.5418 Å

## BRIEF DESCRIPTION OF THE DRAWINGS

Fig. 1 shows an X-ray diffraction pattern of the crystalline silicate produced in Example 1; and

Fig. 2 shows an X-ray diffraction pattern of the crystalline silicate produced in Example 3. In Figs 1 and 2, $\theta$ means the angle of the incidence.

## DETAILED DESCRIPTION OF THE INVENTION

In accordance with the process of the invention, (a) a silica source, (b) an alumina source, (c) an alkali metal and/or alkaline earth metal source, and (d) methanol are added to water to prepare an aqueous mixture, and the thus-prepared aqueous mixture is reacted.

The silica source (a) as used herein is not critical, and silica powder, silicid acid, colloidal silica, dissolved silica, and the like can be used. As the dissolved silica, for example, water glass silicic acid salts and alkaline earth metal silicic acid salts, each containing from 1 to 5 moles of $SiO_2$ per mole of $Na_2O$ or $K_2O$, can be used.

As the alumina source (b), various compounds can be used, including aluminum sulfate, sodium aluminate, colloidal alumina, and alumina.

Although the ratio of silica to alumina in the aqueous mixture can be determined appropriately, it is preferred that the molar ratio of silica ($SiO_2$) to alumina ($Al_2O_3$) is at least 10:1, the optimum molar ratio being from 40:1 to 1,000:1.

As the alkali metal and/or alkaline earth metal source (c), various compounds can be used. Compounds which can be used as the alkali metal source include sodium hydroxide and potassium hydroxide. In addition, sodium silicate and sodium aluminate can be used, which serve also as a silica or alumina source. A particularly preferred alkali metal is sodium. Compounds which can be used as the alkaline earth metal source include calcium nitrate and calcium chloride.

The molar ratio of alkali metal and/or alkaline earth metal to silica should be determined within the range of from 0.1:1 to 3:1, with the range of from 0.2:1 to 1:1 being particularly preferred.

Methanol (d) plays an important role in forming the crystal structure of the present crystalline silicate although it does not remain therein as a constitutive component. It is preferred to use a relatively large amount of methanol. Thus, the molar ratio of methanol to water should be determined within the range of from 0.1:1 to 10:1, with the range of from 0.2:1 to 5:1 being particularly preferred, and the molar ratio of methanol to silica should be determined within the range of from 1:1 to 100:1, with the range of from 3:1 to 60:1 being particularly preferred.

The above-described components (a), (b), (c) and (d) are added to water to prepare an aqueous mixture, and then reacted. In the reaction system, the molar ratio of hydroxyl groups in the aqueous mixture to silica (hydroxyl group/silica) should be controlled within the range of from 0.01:1 to 0.5:1. ~~Furthermore, the molar ratio of hydroxyl groups to alumina (hydroxyl group/alumina) should be controlled within the range of 100:1 or more.~~

The aqueous mixture is reacted by heating under the conditions, e.g., temperature and time, that are required for the formation of the desired crystalline silicate. In more detail, it is sufficient to heat the aqueous mixture at a temperature of from 100 to 300°C, preferably 120 to 200°C for a period of from 5 hours to 10 days, preferably from 10 hours to 5 days. The pressure under which the reaction is performed is not critical, and it is usually carried out under autogenous pressure. Moreover, the reaction is usually carried out while stirring, and if necessary, may be performed in an inert gas atmosphere.

It is required for the crystallization reaction to be performed always in the presence of methanol. Unless this requirement is satisfied, the desired crystalline silicate cannot be produced.

After the crystallization reaction, the reaction products are washed with water and dried at a temperature of about 120°C. Thus, there is produced the desired crystalline silicate having the composition represented by the general formula (I) and main X-ray diffraction pattern shown in Table 1 (both being determined after calcining in air at 550°C).

The relative intensities of lattice spacings (d) other than the main lattice spacings shown in Table 1 are not critical in the invention. In particular, however, those crystalline silicates having an X-ray diffraction

pattern as shown in Table 2 are preferred.

Table 2

| Lattice Spacing d (Å) | Relative Intensity* |
|---|---|
| 10.89 ± 0.2 | strong |
| 8.74 ± 0.2 | medium |
| 6.94 ± 0.15 | medium |
| 5.43 ± 0.15 | weak |
| 4.58 ± 0.15 | weak |
| 4.37 ± 0.1 | very strong |
| 4.11 ± 0.1 | weak |
| 3.68 ± 0.1 | very strong |
| 3.62 ± 0.07 | strong |
| 3.46 ± 0.07 | strong |
| 3.34 ± 0.07 | weak |
| 3.30 ± 0.07 | weak |
| 3.22 ± 0.07 | weak |
| 2.98 ± 0.07 | weak |
| 2.94 ± 0.07 | weak |
| 2.90 ± 0.07 | weak |
| 2.78 ± 0.05 | weak |
| 2.74 ± 0.05 | weak |
| 2.71 ± 0.05 | weak |
| 2.52 ± 0.05 | medium |
| 2.43 ± 0.05 | weak |
| 2.37 ± 0.05 | weak |

Irradiation: Cu - $K_\alpha$

Wavelength: 1.5418 Å

* Relative Intensity is evaluated on the basis of the intensity in 4.37 ± 0.1 Å of Lattice Spacing (d).

very strong : 70 - 100 %
strong : 40 - 70 %
medium : 15 - 40 %
weak : 0 - 15 %

The crystalline silicate (ISI-1) of the invention is a silicate having a novel crystal structure, and can be

used effectively as a solid acid catalyst in various reactions. For example, the crystalline silicate (ISI-1) can be used as a catalyst which enables to produce paraxylene through the methylation of toluene by a simplified procedure and furthermore, at a high selectivity over a long period of time. In addition, it can be used as an effective catalyst for efficiently producing an aromatic component-rich gasoline fraction from those hydrocarbons having a low aromatic component content.

In the production of paraxylene by the methylation of toluene, methanol, dimethyl ether, methyl chloride, methyl bromide, methyl iodide, and the like can be used as a methylating agent. Of these compounds, methanol is preferred. This methylating agent and toluene are reacted in the presence of the crystalline silicate catalyst, usually under the conditions: pressure, from atmospheric pressure to 100 kilograms per square centimeter gauge ($kg/cm^2G$), preferably from atmospheric pressure to 50 kilograms per square centimeter gauge; temperature, from 300 to 800°C, preferably 300 to 500°C; and liquid hour space velocity (LHSV), from 0.5 to 5 per hour ($hr^{-1}$), preferably 1 to 2.5 per hour. The molar ratio of toluene to the methylating agent is from 0.5:1 to 10:1 and preferably from 2:1 to 5:1.

The crystalline silicate (ISI-1) of the invention can also be used in the production of an aromatic component-rich gasoline fraction from a hydrocarbon feed having a low aromatic component content. The aromatic component content of the hydrocarbon feed is not critical, and various hydrocarbons having varied aromatic component contents can be used. Usually a hydrocarbon feed having an aromatic component content of 15% by weight or less is used. Typical examples are a gaseous hydrocarbon fraction containing from 2 to 4 carbon atoms, a liquid hydrocarbon fraction, e.g., naphtha, and a mixture thereof.

This reaction is usually performed under the conditions: pressure, atmospheric pressure to 50 kilograms per square centimeter gauge, preferably atmospheric pressure to 20 kilograms per square centimeter gauge; temperature, 200 to 550°C, preferably 350 to 450°C; and weight hour space velocity (WHSV), 0.1 to 50 per hour, preferably 0.5 to 10 per hour.

Accordingly, the crystalline silicate (ISI-1) of the invention can be used widely in the general chemical industry, particularly in petroleum refinery.

The present invention is explained in greater detail by reference to the following examples.

## EXAMPLE 1

The following solutions were prepared.

Solution (A): consisting of 7.52 grams of aluminum sulfate (18 hydrates), 17.6 grams of sulfuric acid (97%), and 100 milliliters of water.

Solution (B): consisting of 211 grams of water glass ($SiO_2$, 29.0% by weight; $Na_2O$, 9.4% by weight; and water, 61.6% by weight), and 46 milliliters of water.

Solution (C): 100 milliliters of water
Solution (D): 376 milliliters of methanol

To Solution (C), Solutions (A) and (B) were gradually added dropwise at the same time, and the resulting mixture was then adjusted to pH 8.5. In addition, Solution (D) was added thereto and mixed. The thus-prepared mixture was placed in a 1-liter autoclave and reacted with stirring at 170°C and autogenous pressure for 20 hours.

The reaction mixture was cooled, and the product was then washed five times with 1.5 liters of water. Thereafter, the solids separated were dried at 120°C for 6 hours to obtain 55.0 grams of a crystalline silicate. This crystalline silicate was calcined in air at 550°C. With the thus-calcined crystalline silicate, the composition (molar ratio) was $0.7(Na_2O)\cdot(Al_2O_3)\cdot 68.5(SiO_2)$, and the X-ray diffraction pattern is as shown in Fig. 1.

EXAMPLE 2

A crystalline silicate was produced in the same manner as in Example 1 except that the amount of aluminum silicate (18 hydrates) added was changed to 18.8 grams.

The composition (molar ratio) of the crystalline silicate which had been calcined in air at 550°C was:

$$1.1(Na_2O)\cdot(Al_2O_3)\cdot 29.5(SiO_2)$$

EXAMPLE 3

(1) Preparation of Catalyst

The following solutions were prepared.

Solution (A): consisting of 11.3 grams of aluminum sulfate (18 hydrates), 17.6 grams of sulfuric acid (97%), and 100 milliliters of water.

Solution (B): consisting of 211 grams of water glass ($SiO_2$, 29.0% by weight; $Na_2O$, 9.4% by weight; and water, 61.6% by weight), and 46 milliliters of water.

Solution (C): 100 milliliters of water
Solution (D): 376 milliliters of methanol

To Solution (C), Solutions (A) and (B) were gradually added dropwise at the same time and mixed, and the resulting mixture was then adjusted to pH 8.5. In addition, Solution (D) was added thereto and mixed. The thus-prepared mixture was placed in a 1-liter autoclave, and reacted with stirred at 170°C and autogenous pressure for 20 hours.

The reaction mixture was cooled, and the product was then washed five times with 1.5 liters of water. Thereafter, the solids which had been filteres off was dried at 120°C for 6 hours to obtain 55.0 grams of a crystalline silicate. The composition (molar ratio) of the crystalline silicate which had been calcined in air at 550°C was:

$$0.8(Na_2O)\cdot(Al_2O_3)\cdot 46.7(SiO_2)$$

The X-ray diffraction pattern is shown in Fig. 2. It can be seen from Fig. 2 that the above-produced crystalline silicate falls within the scope of the invention.

0.5N hydrochloric acid was added to the crystalline silicate in a proportion of 5 milliliters per gram of the crystalline silicate, and the crystalline silicate was treated under reflux for 6 hours. Water-washing and filtration were repeated until any chlorine ion was not detected in the water phase. The thus-treated crystalline silicate was dried at 120°C, and then calcined at 550°C for 6 hours. Thereafter, alumina sol corresponding to 20% by weight of alumina was added thereto, and the resulting mixture was extrusion-molded. The mold was dried at 120°C for 3 hours and subsequently, at 550°C for 6 hours in air.

(2) Methylation of Toluene

The crystalline silicate catalyst produced in (1) above was placed in a quartz reaction tube. A mixture of toluene and methanol (molar ratio: 2:1) was introduced in the quartz reaction tube and reacted under the conditions: pressure, atmospheric pressure; temperature, 400°C; and LHSV, 1.0 per hour. The results are shown in Table 3.

EXAMPLE 4

The methylation of toluene was performed in the same manner as in (2) of Example 3 except that the reaction temperature was changed to 450°C. The results are shown in Table 3.

EXAMPLE 5

The methylation of toluene was performed in the same manner as in (2) of Example 3 except that the molar ratio of toluene to methanol was changed to 4:1. The results are shown in Table 3.

EXAMPLE 6

The methylation of toluene was performed in the same manner as in Example 5 except that the reaction temperature was changed to 450°C. The results are shown in Table 3.

COMPARATIVE EXAMPLE 1

The methylation of toluene was performed in the same manner as in (2) of Example 3 except that ZSM-5 Zeolite was used as a catalyst in place of the crystalline silicate. The results are shown in Table 3.

Table 3

| | Example 3 | Example 4 | Example 5 | Example 6 | Comparative Example 1 |
|---|---|---|---|---|---|
| **Reaction Conditions** | | | | | |
| Toluene/Methanol (molar ratio) | 2:1 | 2:1 | 4:1 | 4:1 | 2:1 |
| Pressure | atmospheric pressure | atmospheric pressure | atmospheric pressure | atmospheric pressure | atmospheric pressure |
| Temperature (°C) | 400 | 450 | 400 | 450 | 400 |
| LHSV ($hr^{-1}$) | 1.0 | 1.0 | 1.0 | 1.0 | 1.0 |
| **Reaction Results** | | | | | |
| Conversion of Toluene (%) | 13.6 | 22.9 | 15.1 | 19.3 | 45.6 |
| Selectivity of Xylene (%) | 84.2 | 76.2 | 87.4 | 80.5 | 53.5 |
| Composition of Xylene (%) | | | | | |
| Para-xylene | 49.3 | 49.4 | 52.3 | 54.9 | 24.4 |
| Meta-xylene | 16.1 | 21.5 | 14.5 | 18.3 | 53.1 |
| Ortho-xylene | 34.6 | 29.1 | 33.2 | 26.8 | 22.5 |

EXAMPLE 7

Reaction of Hydrocarbon

The crystalline silicate catalyst produced in (1) of Example 3 was placed in a flow type reactor. A hydrocarbon feed having the composition shown in Table 4 was introduced and reacted under the conditions: pressure, atmospheric pressure; temperature, 400°C; and WHSV, 0.8 per hour. The results are shown in Table 5.

EXAMPLE 8

(1) Preparation of Catalyst

The crystalline silicate powder produced in Example 1 was ion-exchanged twice with 1N ammonium nitrate added in a proportion of 5 milliliters per gram of the powder, and subsequently, ion-exchanged twice with 0.5N zinc sulfate added in a proportion of 10 milliliters per gram of the powder. The thus-treated crystalline silicate powder was fully washed with ion-exchanged water and filtered off, and thereafter, dried at 120°C and further calcined in air at 550°C for 6 hours. To the thus-calcined powder was added alumina sol corresponding to 20% by weight of alumina, and the resulting mixture was extrusion-molded. The mold was dried at 120°C for 3 hours and subsequently, calcined in air at 550°C for 6 hours to obtain a zinc ion exchanged type catalyst.

(2) Reaction of Hydrocarbon

The reaction of hydrocarbon was performed in the same manner as in Example 7 except that the reaction temperature was changed to 450°C and, as a catalyst, the crystalline silicate catalyst produced in (1) above was used. The results are shown in Table 5.

EXAMPLE 9

The reaction of hydrocarbon was performed in the same manner as in Example 7 except that the reaction pressure was changed to 10 kilograms per square centimeter gauge. The results are shown in Table 5.

Table 4

| Component | Proportion (% by weight) |
|---|---|
| Ethane | 0 |
| Ethylene | 0 |
| Propane | 0.2 |
| Propylene | 0.1 |
| iso-Butane | 38.0 |
| n-Butane | 6.7 |
| 1-Butene | 14.7 |
| iso-Butene trans-2-Butene | 33.4 |
| cis-2-Butene | 6.9 |
| $C_5^+$ | 0.1 |

Table 5

| | Example 7 | Example 8 | Example 9 |
|---|---|---|---|
| Reaction Conditions | | | |
| Pressure ($kg/cm^2$ G) | atmospheric pressure | atmospheric pressure | atmospheric pressure |
| Temperature (°C) | 400 | 450 | 400 |
| WHSV ($hr^{-1}$) | 0.8 | 0.8 | 0.8 |
| Conversion of Butene (%) | 69.4 | 80.8 | 100 |
| Reaction Results | | | |
| Methane (% by weight) | 0.0 | 0.2 | 0.0 |
| Ethane + Ethylene (% by weight) | 0.4 | 1.5 | 0.3 |
| $C_3$ (Propylene) (% by weight) | 9.0 (7.0) | 10.3 (8.3) | 4.2 (1.8) |
| $C_4$ (Butene) (% by weight) | 77.9 (15.3) | 78.9 (9.8) | 65.5 (3.3) |
| $C_5^+$ (% by weight) | 12.8 | 9.0 | 30.0 |
| Aromatic Component/$C_5^+$ (% by weight) | 27.7 | 31.7 | 52.3 |

What is claimed is:

1. A crystalline silicate which has a composition represented by the general formula (I) after being calcined in air at 550°C,

$$p(M_{2/n}O)\cdot(Al_2O_3)\cdot q(SiO_2) \qquad \text{------ (I)}$$

(Wherin M represents an alkali metal and/or alkaline earth metal, n represents the atomic valency of M, and p and q represent molar ratios and are $0.3 \leqq p \leqq 3.0$, and $q \geqq 10$), and which has X-ray diffraction data as shown below:

| Lattice Spacing d (Å) | Relative Intensity |
|---|---|
| 10.89 ± 0.2 | strong |
| 8.74 ± 0.2 | medium |
| 6.94 ± 0.15 | medium |
| 5.43 ± 0.15 | weak |
| 4.58 ± 0.15 | weak |
| 4.37 ± 0.1 | very strong |
| 4.11 ± 0.1 | weak |
| 3.68 ± 0.1 | very strong |
| 3.62 ± 0.07 | strong |
| 3.46 ± 0.07 | strong |
| 3.34 ± 0.07 | weak |
| 3.30 ± 0.07 | weak |
| 3.22 ± 0.07 | weak |
| 2.98 ± 0.07 | weak |
| 2.94 ± 0.07 | weak |
| 2.90 ± 0.07 | weak |
| 2.78 ± 0.05 | weak |
| 2.74 ± 0.05 | weak |
| 2.71 ± 0.05 | weak |
| 2.52 ± 0.05 | medium |
| 2.43 ± 0.05 | weak |
| 2.37 ± 0.05 | weak |

European Patent Office

# EUROPEAN SEARCH REPORT

0170003
Application number

EP 85 10 6706

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | US-A-4 199 556 (PLANK et al.) --- | | C 01 B 33/28<br>B 01 J 29/00 |
| A | US-A-3 516 786 (MAHER et al.) --- | | |
| A | GB-A-1 553 209 (ICI LTD.) ----- | | |
| | | | TECHNICAL FIELDS SEARCHED (Int. Cl 4) |
| | | | C 01 B 33/28<br>B 01 J 29/28 |

The present search report has been drawn up for all claims

| Place of search | Date of completion of the search | Examiner |
|---|---|---|
| BERLIN | 19-09-1985 | CLEMENT J.P. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82